# EUROPÄISCHE PATENTSCHRIFT

(11) **EP 0 708 811 B1**
(45) Veröffentlichungstag und Bekanntmachung des Hinweises auf die Patenterteilung: **12.01.2000**
(21) Anmeldenummer: 94922263.2
(22) Anmeldetag: 06.07.1994
(51) Int. Cl.: C10M 107/34, C10M 105/38, C10M 101/04, C10N 40/08

(54) **GRUNDÖL AUF TRIGLYCERIDBASIS FÜR HYDRAULIKÖLE**
BASE OIL MADE FROM TRIGLYCERIDES FOR USE IN HYDRAULIC FLUIDS
HUILE DE BASE CONSTITUEE DE TRIGLYCERIDES POUR HUILES HYDRAULIQUES

(30) Priorität: 15.07.1993 DE 4323771
(43) Veröffentlichungstag der Anmeldung: 01.05.1996
(73) Patentinhaber: Cognis Deutschland GmbH, 40589 Düsseldorf (DE)
(72) Erfinder: KLEIN, Johann, D-40233 Düsseldorf (DE); BONGARDT, Frank, D-40591 Düsseldorf (DE); DAUTE, Peter, D-27616 Beverstedt (DE); FIES, Matthias, D-47800 Krefeld (DE)
(86) Internationale Anmeldenummer: EP9402212
(87) Internationale Veröffentlichungsnummer: WO9502659

(56) Entgegenhaltungen:
- EP-A- 0 247 509
- EP-A- 0 325 010
- EP-A- 0 458 584
- EP-A- 0 602 981
- WO-A-88/05808
- GB-A- 1 047 253
- GB-A- 1 050 497
- GB-A- 1 210 509
- US-A- 3 871 837

## Beschreibung

Die Erfindung betrifft ein biologisch abbaubares Grundöl auf Triglyceridbasis, seine Herstellung, sowie dieses Grundöl enthaltende Hydrauliköle.

In der Vergangenheit aber auch noch bis heute wird der überwiegende Teil der Hydrauliköle aus Mineralöl hergestellt. In Einsatzgebieten, in denen beispielsweise durch nicht zu verhindernde Leckagen Ölaustritte in die Umwelt als Möglichkeit einzubeziehen sind, wird zunehmend der Einsatz von Hydraulikölen gefordert, die als Ölbasis umweltfreundliche Esteröle, insbesondere solche auf Basis Rüböl und/oder Sojaöl enthalten. Typische Einsatzgebiete des hier betroffenen Bereiches sind die Maschinen und Arbeitsgeräte der Forstwirtschaft, der Landwirtschaft, Bauwirtschaft und dergleichen. Gefordert wird hier heute der Einsatz von Hydraulikölen der Wassergefährdungsklasse 0-1. Hydrauliköle auf Esterbasis sind geeignet, diesen Anforderungen zu entsprechen.

Die für den praktischen Einsatz wesentlichen Esteröle der zuvor genannten Art, d.h. Öle auf Basis gereinigter, insbesondere von Amylopektinen und anderen Schleimstoffen befreiter Rüböle und/oder Sojaöle zeigen im praktischen Einsatz jedoch 2 entscheidende Schwächen:

Die auf Basis mehrfach ungesättigter Fettsäuresysteme aufgebauten Esteröle neigen auch bei nur mäßig erhöhten Betriebstemperaturen von beispielsweise 50 bis 80°C zur raschen Verdickung. Anlaß hierfür ist die Bereitschaft der olefinischen Doppelbindungen der esterbildenden Säuren des hier betroffenen Öltyps, unter Einfluß von Luftsauerstoff Reaktionen einzugehen, die letztlich zur Viskositätszunahme führen. Zwar ist grundsätzlich bekannt, daß gerade in Hydraulikölen durch Zusatz von Antioxidantien solche unerwünschten Viskositätssteigerungen zu dämpfen sind, es zeigt sich jedoch, daß die bisher in Hydraulikölen auf Mineralölbasis bevorzugt eingesetzten Antioxidantien in Esterölen der hier betroffenen Art nur eine unzureichende Leistung erbringen.

Eine weitere wichtige Einschränkung für Hydrauliköle auf Basis der genannten umweltfreundlichen Esteröle ist die unzureichende Kältestabilität. Gereinigtes Rüböl besitzt beispielsweise einen Erstarrungs- bzw. Stockpunkt von -16°C. Schon vor Erreichen des Erstarrungspunktes ist mit sinkenden Temperaturen ein bemerkenswerter Anstieg der Viskosität gegeben. Der beispielsweise im Winterbetrieb doch vergleichsweise hoch liegende Erstarrungspunkt des Rüböls bringt für den praktischen Einsatz der Hydrauliköle bei niederen Umgebungstemperaturen beträchtliche Probleme. Verständlicherweise kann diese Problematik im Betrieb dramatisch dadurch verstärkt werden, daß über eine oxidative Eindickung des Esteröls der zuvor geschilderten Art gleichzeitig auch die erhebliche Heraufsetzung des Erstarrungspunktes des Hydrauliköls ausgelöst wird. Der Zusatz von Stockpunktserniedrigern gibt hier keine Lösung des technischen Problems. Es ist bekannt, daß die Wirkung solcher Pourpoint-Depressants bei längerem Verweilen im zu behandelnden Öl verschwindet.

Die DE-A-39 27 155 beschreibt ein umweltfreundliches Grundöl auf Naturstoffbasis für die Formulierung von Hydraulikölen enthaltend ein Rüb- und/oder Sojaöl als Ölhauptkomponente, speziell ausgewählten Antioxidantien und einen zur Ölhauptkomponentenmenge gleichen Anteil von Estern des Trimethylolethans, Trimethylolpropans und/oder des Neopentylalkohols mit C₅-C₁₀-Monocarbonsäuren oder zumindest teilweise ungesättigten Fettsäuren auf Rüböl-, Sojaöl- oder Sonnenblumenölbasis.

In GB-B-1 047 253 werden Reaktionsprodukte von Ethylenoxid und Propylenoxid mit Ricinusöl als geeignete Bestandteile Kältestabiler Hydrauliköle offenbart.

Der vorliegenden Erfindung liegt als eine Aufgabe zugrunde, Grundöle für die Formulierung von Hydraulikölen zur Verfügung zu stellen, die bereits von sich aus und ohne Zusatz von Pourpoint-Depressants oder Beimischung von synthetischen Estern eine hohe Kältestabilität aufweisen.

Diese Aufgabe wurde erfindungsgemäß gelöst durch Grundöle auf Triglyceridbasis erhältlich durch
a) Ethoxylierung und/oder Propoxylierung von Glycerin mit 0,5 bis 3 mol Ethylenoxid (E0) und/oder Propylenoxid (P0) und anschließender Veresterung mit gesättigten und/oder ungesättigten Fettsäuren mit 6 bis 24 Kohlenstoffatomen aus natürlichen Quellen oder Gemischen davon in an sich bekannter Weise oder
b) Insertion von 0,5 bis 3 mol E0 und/oder P0 in natürliche Öle oder Fette, außer Ricinusöl, in an sich bekannter Weise, in Abmischung mit Antioxidantien sowie gegebenfalls weiteren, üblichen Zusatzstoffen.

Vorzugsweise werden für die Ethoxylierung und/oder Propoxylierung von Glycerin 0,5 bis 2 mol, besonders bevorzugt 0,5 bis 1 mol E0 und/oder P0 eingesetzt.

Für die Insertionsreaktion von E0 und/oder P0 in die natürlichen Öle oder Fette werden vorzugsweise 0,5 bis 2 mol, besonders bevorzugt 0,5 bis 1 mol E0 und/oder P0 eingesetzt.

Als Fettsäurekomponente für die Veresterung der Umsetzungsprodukte von E0 und/oder P0 mit Glycerin kommen die gesättigten oder ungesättigen Fettsäuren mit 6 bis 24 Kohlenstoffatomen aus natürlichen Quellen in Frage.

Besonders bevorzugt sind als gesättigte Fettsäuren die Hexansäure (Capronsäure), Octansäure (Caprylsäure), Decansäure (Caprinsäure), Dodecansäure (Laurinsäure), Tetradecansäure (Myristinsäure), Hexadecansäure (Palmitinsäure), Octadecansäure (Stearinsäure), Eicosansäure (Arachinsäure), Docosansäure (Behensäure) oder die Tetracosansäure (Lignocerinsäure).

Besonders bevorzugt sind als ungesättigte Fettsäuren die Tetradecensäure (Myristoleinsäure), Hexadecensäure (Palmitoleinsäure), Octadecensäure (Ölsäure), Eicosensäure (Gadoleinsäure), Docosensäure (Erucasäure), 12-Oxy-octadecensäure (Ricinolsäure), Octadecadiensäure (Linolsäure), Octadecatriensäure (Linolensäure), Eicosatetraensäure (Arachidonsäure) oder Docosapentaensäure (Clupanodonsäure).

Für die Insertionsreaktion von E0 und/oder P0 in die natürlichen Öle oder Fette werden Kokosöl, Palmkernöl, Palmöl, ErdnuBöl, Baumwollöl, Sojaöl, Sonnenblumenöl, erucasäurereiches und erucasäurearmes Rapsöl (Rüböl), Talg und Fischöl bevorzugt.

Das Verfahren zur Ethoxylierung und/oder Propoxylierung von Glycerin mit Ethylenoxid und/oder Propylenoxid ist an sich bekannt. Es kann daher auf einschlägige Fachliteratur verwiesen werden.

Allgemein stellen Anlagerungsprodukte von Alkylenoxiden an Glycerin bekannte Stoffe dar, die nach den einschlägigen Verfahren der präparativen organischen Chemie erhalten werden können. Großtechnisch erfolgt ihre Herstellung durch Ethoxylierung bzw. Propoxylierung von Glycerin in Gegenwart basischer Katalysatoren, wie beispielsweise Lithiumhydroxid, Kaliumhydroxid, Natriummethylat, Strontiumphenolat oder caliciniertem Hydrotalcit, bei Temperaturen im Bereich von 120-180°C und einem Druck von 1 bis 5 bar. Werden gemischte Alkoxylate eingesetzt, so kann die Alkoxylierung blockweise oder randomisiert erfolgen. Im Anschluß an die Alkoxylierung können die Produkte durch Zugabe von Säuren (Phosphorsäure, Essigsäure, Milchsäure) neutralisiert werden. Nicht neutralisierte Katalysatoren -insbesondere Lithiumhydroxid- können jedoch auch für den nachfolgenden Reaktionsschritt der Veresterung weiterverwendet werden.

Die sich an die Ethoxylierung und/oder Propoxylierung von Glycerin anschließende Veresterung des Reaktionsproduktes mit den oben genannten 6 bis 24 Kohlenstoffatomen enthaltenden gesättigten oder ungesättigten Fettsäuren erfolgt ebenfalls in für den Fachmann an sich bekannter Weise (z.B. Synthetic Fats von P.N. Williams in "Chemistry and Industry" (London) 19 (1947), 253).

Das Verfahren zur Insertion von Alkylenoxiden und insbesondere von Ethylenoxid und/oder Propylenoxid ist in der gängigen Fachliteratur ebenfalls eingehend beschrieben und verläuft in Gegenwart von Katalysatoren wie insbesondere Lithiumhydroxid, calciniertem Hydrotalcit, Kaliumhydroxid oder Natriumalkoxylat. Im einzelnen wird hierzu auf die EP-A-0 247 509 verwiesen, wo die Insertion von Alkylenoxiden in Triglyceride durch weitere Bezugnahmen auf den Stand der Technik detailliert beschrieben ist.

Durch das Verfahren der Ethylenoxid- und/oder Propylenoxidinsertion in natürliche Öle oder Fette kann somit unmittelbar der Pourpoint der zugrundeliegenden Öle/Fette erheblich gesenkt werden. Mit diesem Verfahren ist es möglich, preisgünstige Hydraulikgrundöle herzustellen, die chemisch gegenüber den zugrundeliegenden natürlichen Ölen und Fetten nur geringfügig verändert werden müssen, um hinsichtlich ihrer Kältestabilität beachtlich verbessert werden zu können. Überraschend ist hierbei jedoch, daß sich die Viskosität der ethoxylierten und/oder propoxylierten Produkte kaum von der, der zugrundeliegenden Öle/Fette unterscheidet, sie in der Regel sogar erhöht ist.

Da sich die Verfahren zur Herstellung der erfindungsgemäßen Grundöle aus einzelnen, an sich bekannten Verfahrensschritten zusammensetzen, erübrigen sich hier weitere Ausführungen hierzu.

Die nach den oben genannten Verfahren erhältlichen Öle können als Grundöle für die Formulierung von Hydraulikölen verwendet werden. Diese Grundöle zeigen ein besonders gutes Kälteverhalten, da sie durch einen besonders niedrigen Pourpoint (Titer) gekennzeichnet sind. Sie sind ferner gut biologisch und schnell abbaubar (CEC L33 T82-Test, ≥ 80%).

Die vorliegende Erfindung betrifft ferner Hydrauliköle, welche neben den üblichen Zusatzstoffen die oben genannten Grundöle enthalten. Die Hydrauliköle sind erhältlich durch intensives Vermischen der Grundöle mit den üblichen Zusatzstoffen. Dem Fachmann sind hierzu aus der einschlägigen Literatur geeignete Verfahren bekannt.

Die erfindungsgemäßen Hydrauliköle enthalten wenigstens 90 Gew.-%, vorzugsweise wenigstens 95 Gew.-% und besonders bevorzugt wenigstens 98 Gew.-% eines der oben genannten Grundöle oder eines Gemisches davon.

Die erfindungsgemäßen Grundöle und damit auch die sie enthaltenden Hydrauliköl-Zusammensetzungen weisen eine hohe Kältestabilität auf, so daß für übliche Anwendungen ein Zusatz von zusätzlichen Pourpoint-Depressants oder eine Beimischung von synthetischen Estern nicht notwendig ist. Die erfindungsgemäßen Grundöle zeigen gegenüber den ihnen zugrundeliegenden Fetten/Ölen einen stark erniedrigten Pourpoint, ohne daß die Viskosität im Vergleich zu der, der zugrundeliegenden Fette/Öle beeinträchtigt wird.

Zur Formulierung des fertigen Hydrauliköls aus dem Grundöl bedarf es in bekannter Weise des Zusatzes weiterer, üblicher Komponenten. So können den erfindungsgemäß beschriebenen Grundölen insbesondere Antioxidantien, Korrosionsinhibitoren, Hochdruckadditive, Verschleißschutzadditive oder, sofern notwendig, weitere Pourpoint-Depressants zugesetzt werden.

Bei den Hochdruckadditiven handelt es sich insbesondere um geschwefelte Triglyeride, geschwefelte Fettsäurealkylester, geschwefelte Spermöle, Phosphorsäureester wie Trioleylalkoholphosphat oder Triarylphosphat.

Als Verschleißschutzadditive sind insbesondere Zinkdialkyldithiophosphatverbindungen wie Zink(di-2-ethylhexyldithiophosphat) geeigent.

Als Pourpoint-Depressant sind beispielsweise die unter den Handelsnamen "Edenor" 2410 (Handelsprodukt der Firma Henkel KGaA, Düsseldorf) und Viskoplex-Typen (Handelsprodukte der Firma Röhm, Darmstadt) vertriebenen Produkte zu erwähnen. Bei diesen Produkten handelt es sich um Pourpoint-Depressants auf Polymerbasis, die die Kristallisation unendlich verzögern.

Als Korrosionsschutzinhibitoren sind insbesondere Bernsteinsäurehalbester, Sorbitanmonooleat, Aminseifen von langkettigen Fettsäuren geeignet. Erwähnt seien in diesem Zusammenhang Additin (Handelsprodukt der Firma Rheinchemie, Mannheim) Edenor oder Eumulgin (Handelsprodukte der Firma Henkel KGaA, Düsseldorf).

Als Antioxidantien eignen sich insbesondere Kombinationen aus sterisch gehinderten Aromaten, insbesondere TBHQ (tertiärbutylhydroxy-Chinolin) oder BHT (butoxyliertes Hydroxytoluol) und anionische Antioxidantien, wie insbesondere BHA (butoxyliertes Hydroxyanisol) oder Phenothiazin (Handelsprodukt der Firma Hoechst AG, Frankfurt). Weiterhin kann ein flüssiges Antioxidants - Edenor VP 2465 (Handelsprodukt der Firma Henkel KGaA, Düsseldorf) - verwendet werden, das aus einer Kombination von anionischem und phenolischem Antioxidants besteht.

Im übrigen wird zu der speziellen Formulierung von Hydraulikölen, ihren Zusatzstoffen und den Mengen dieser Zusatzstoffe auf den eingehend referierten druckschriftlichen Stand der Technik dieser Materialklasse verwiesen.

Die vorliegende Erfindung wird durch die nachfolgenden Beispiele erläutert, ohne jedoch darauf beschränkt zu sein.

### Beispiele:

### 1. Herstellung von Glycerinpropoxylat-Trioleat

### a) Glycerinpropoxylat

In einem 4-l-Stahlautoklaven wurden 2400 g (26 Mol) Glycerin vorgelegt, mit 0,45 g Lithiumhydroxid versetzt und auf 80°C erhitzt. Durch 5-maliges Evakuieren und Belüften mit Stickstoff wurden anhaftende Wasserspuren entfernt. Danach wurde die Reaktionsmischung auf 150°C erhitzt und 1500 g (26 Mol) Propylenoxid portionsweise zudosiert, so daß der Druck im Reaktor einen Wert von 5 bar nicht überstieg. Nach Beendigung der Reaktion (ca. 3 h) wurde auf 80 bis 100°C abgekühlt und zur Entfernung von Spuren nichtumgesetzten Propylenoxids ca. 15 min evakuiert. Es wurden ca. 3900 g Glycerinpropoxylat in Form einer klaren, farblosen Flüssigkeit erhalten, die eine Hydroxylzahl von 1200 aufwies.

### b) Glycerinpropoxylat-Trioleat

Eine Mischung aus 157.2 g (1.04 Mol) Glycerinpropoxylat mit einer OHZ von 1113 und 833.2 g (3 Mol) einer technischen Ölsäure mit einer SZ von 203 und einer Iodzahl von 90 wurde in Anwesenheit von 1 g Zinnoxalat 2 Stunden aus 240°C erhitzt. Das entstandene Kondensationswasser wurde abdestilliert. Anschließend wurde weiter 5 Stunden bei 240°C erhitzt und das Kondensationswasser in einem Vakuum von 16 mbar abdestilliert. Danach wurden zur vollständigen Veresterung der Carbonsäure weitere 2.5 g (0.017 Mol) Glycerinpropoxylat mit einer OHZ von 1113 zugegeben und 3 Stunden bei 240°C und einem Vakuum von 16 mbar erhitzt. Anschließend wurde auf 95°C abgekühlt, 20 g Bleicherde (Montmorillonit-Basis) zugegeben und abfiltriert. Das Produkt hat eine dunkel-gelbe Farbe und ist leicht zähflüsssig. Die OHZ ist 8.9, die SZ 0.9.

### c) Umsetzung von Rüböl mit Propylenoxid (Insertion)

In einem 2-l-Stahlautoklaven wurde eine Mischung aus 872 g (1 Mol) Rüböl neuer Züchtung und 8,7 g (95 mmol) Glycerin mit 0,94 g Lithiumhydroxidhydrat versetzt und auf 100°C erhitzt. Durch 5-maliges Evakuieren und Belüften mit Stickstoff wurden anhaftende Wasserspuren entfernt. Danach wurde die Reaktionsmischung auf 180°C erhitzt und 58 g (1 Mol) Propylenoxid portionsweise zudosiert, so daß der Druck im Reaktor einen Wert von 5 bar nicht überstieg.

Nach Beendigung der Reaktion (ca 3 h) wurde auf 80 bis 100°C abgekühlt und zur Entfernung von Spuren nichtumgesetzten Propylenoxids ca. 15 min. evakuiert. Nach Neutralisation des Katalysators mit 2,24 g 90 proz. Milchsäure und Filtration erhielt man ca. 930 g einer gelben Flüssigkeit.

### 2. Vergleich von Glycerinmonopropoxylat (GPO)-Trioleat mit Rüböl

1 Mol Glycerin wird mit 1 Mol Propylenoxid umgesetzt. Das daraus entstandene Glycerinmonopropoxylat (GP0) wurde mit 3 Mol der Fettsäure Edenor TiO5 (Ölsäure ex Rindertalg) verestert. Das Produkt wurde mit Rüböl verglichen:

| | GP0-trioleat | Rüböl *⁾ |
|---|---|---|
| Säurezahl (DIN 53 402) | 2,4 | 1 |
| | | |
| Kinemat. Viskosität [mm²] (DIN 51 562) | | |
| bei 40°C: | 43,8 | 38,1 |
| bei 100°C | 8,98 | 8,51 |
| | | |
| Viskositätsindex (DIN ISO 2909) | 192 | 210 |
| | | |
| Demulgiervermögen [min.]: (DIN 51 599) | > 60 | 8 |
| | | |
| Luftabscheidevermögen [min.]: | | |
| (DIN 51 381) | 7 | 6 |
| | | |
| Cloudpoint [°C]: (DIN ISO 3015) | -21 | -16 |
| | | |
| Pourpoint [°C] | -34 | -17 |

| | | |
|---|---|---|
| *) erucaarmes, raffiniertes Rapsöl | | |

## Patentansprüche

1. Verwendung des Produkts
a) einer Ethoxylierung und/oder Propoxylierung von Glycerin mit 0.5 bis 3 mol Ethylenoxid und/oder Propylenoxid und anschließender Veresterung mit gesättigten und/oder ungesättigten Fettsäuren mit 6-24 Kohlenstoffatomen aus natürlichen Quellen oder Gemischen davon auf an sich bekannte Weise oder
b) einer Insertion von 0.5 bis 3 mol E0 und/oder P0 in natürliche Öle oder Fette, außer Ricinusöl, in an sich bekannter Weise,
in Abmischung mit Antioxidantien sowie gegebenenfalls weiteren, üblichen Zusatzstoffen als Hydrauliköl.

2. Verwendung nach Anspruch 1, dadurch gekennzeichnet, daß a) oder b) einen Anteil von wenigstens 90 % im Hydrauliköl haben.

3. Hydrauliköl, enthaltend das Produkt
a) einer Ethoxylierung und/oder Propoxylierung von Glycerin mit 0.5 bis 3 mol Ethylenoxid und/oder Propylenoxid und anschließender Veresterung mit gesättigten und/oder ungesättigten Fettsäuren mit 6-24 Kohlenstoffatomen aus natürlichen Quellen oder Gemischen davon auf an sich bekannte Weise oder
b) einer Insertion von.0.5 bis 3 mol E0 und/oder P0 in natürliche Öle oder Fette, außer Ricinusöl, in an sich bekannter Weise,
Antioxidantien sowie gegebenenfalls weitere, übliche Zusatzstoffe.

4. Hydrauliköl nach Anspruch 3, dadurch gekennzeichnet, daß es als übliche Zusatzstoffe Korrosionsinhibitoren, Hochdruckadditive und/oder Pourpoint-Depressants enthält.

## Claims

1. The use of the product of
a) the ethoxylation and/or propoxylation of glycerol with 0.5 to 3 moles of ethylene oxide and/or propylene oxide and subsequent esterification with saturated and/or unsaturated C₆₋₂₄ fatty acids from natural sources or mixtures thereof by methods known per se or
b) the insertion of 0.5 to 3 moles of EO and/or PO into natural oils or fats, except castor oil, by methods known per se
in admixture with antioxidants and optionally other typical additives as a hydraulic oil.

2. The use claimed in claim 1, characterized in that a) or b) makes up at least 90% of the hydraulic oil.

3. A hydraulic oil containing the product of
a) the ethoxylation and/or propoxylation of glycerol with 0.5 to 3 moles of ethylene oxide and/or propylene oxide and subsequent esterification with saturated and/or unsaturated C₆₋₂₄ fatty acids from natural sources or mixtures thereof by methods known per se or
b) the insertion of 0.5 to 3 moles of EO and/or PO into natural oils or fats, except castor oil, by methods known per se,
antioxidants and optionally other typical additives.

4. A hydraulic oil as claimed in claim 3, characterized in that it contains corrosion inhibitors, high-pressure additives and/or pour point depressants as typical additives.

## Revendications

1. Utilisation comme huile hydraulique du produit :
a) d'une éthoxylation et/ou d'une propoxylation de glycérine avec de 0,5 à 3 moles d'oxyde d'éthylène et/ou d'oxyde de propylène et estérification subséquente avec des acides gras saturés et/ou insaturés ayant 6 à 24 atomes de carbone, issus de sources naturelles, ou de mélanges de ceux-ci, de manière connue en soi, ou
b) d'une insertion de 0,5 à 3 moles d'EO et/ou de PO dans des huiles naturelles ou des graisses, hormis l'huile de ricin, de manière connue en soi,
en mélange avec des antioxydants ainsi que, le cas échéant, d'autres adjuvants usuels.

2. Utilisation selon la revendication 1,
caractérisée en ce que
a) ou b) sont en une proportion d'au moins 90 % dans l'huile hydraulique.

3. Huile hydraulique, contenant le produit :
a) d'une éthoxylation et/ou d'une propoxylation de glycérine avec de 0,5 à 3 moles d'oxyde d'éthylène et/ou d'oxyde de propylène et estérification subséquente avec des acides gras saturés et/ou insaturés ayant 6-24 atomes de carbone, issus de sources naturelles, ou de mélanges de ceux-ci, de manière connue en soi, ou
b) d'une insertion de 0,5 à 3 moles d'EO et/ou de PO dans des huiles naturelles ou des graisses, hormis l'huile de ricin, de manière connue en soi,
en mélange avec des antioxydants ainsi que, le cas échéant, d'autres adjuvants usuels.

4. Huile hydraulique selon la revendication 3,
caractérisée en ce qu'
elle contient, à titre d'adjuvants usuels, des inhibiteurs de corrosion, des additifs pour hautes pressions et/ou des abaisseurs de point de coulée.
